# EUROPEAN PATENT SPECIFICATION

(11) **EP 3 579 737 B1**
(45) Date of publication and mention of the grant of the patent: **11.11.2026**
(21) Application number: 17896021.7
(22) Date of filing: 17.02.2017
(51) Int. Cl.: A61B 3/10, A61B 3/12, A61B 3/00, G01B 9/02

(54) **RETINAL THICKNESS**
RETINADICKE
ÉPAISSEUR RÉTINIENNE

(30) Priority: 13.02.2017 US 201715431150
(43) Date of publication of application: 18.12.2019
(73) Proprietor: Cellview Imaging Inc., North York ON M3K 2A2 (CA)
(72) Inventor: HATHAWAY, Mark, Toronto, Ontario M2N 7E2 (CA); WEITZ, Rishard, Toronto, Ontario M2P 1M5 (CA)
(74) Representative: Marks & Clerk LLP
(86) International application number: PCT/CA2017/050214
(87) International publication number: WO 2018/145192

(56) References cited:
- WO-A1-2016/004508
- WOJTKOWSKI M: "In vivo human retinal imaging by Fourier domain optical coherence tomography", JOURNAL OF BIOMEDICAL OPTICS, SPIE, vol. 7, no. 3, 1 January 2002 (2002-01-01), pages 457 - 463, XP002438097, ISSN: 1083-3668, DOI: 10.1117/1.1482379
- WOJTKOWSKI ET AL.: "In vivo human retinal imaging by Fourier Domain optical coherence tomography", JOURNAL OF BIOMEDICAL OPTICS, vol. 7, no. 3, July 2002 (2002-07-01), pages 457 - 463, XP002272406, Retrieved from the Internet <URL:https://www.researchgate.net/profile/Rainer_Leitgeb/publication/11212503_In_vivo_human_retinal_imaging_by_Fourier_Domain_optical_coherence_tomography/links/55b60f6008aec0e5f436d7db/In-vivo-human-retinal-imaging-by-Fourier-Domain-optical-coherence-tomography.pdf> [retrieved on 20171017]
- WOLFGANG ET AL.: "Ultrahigh-resolution ophthalmic optical coherence tomography", NATURE MEDICINE, vol. 7, no. 4, April 2001 (2001-04-01), pages 502 - 507, XP002251061, Retrieved from the Internet <URL:https://www.ncbi.nlm.nih.gov/pmc/articles/PMC1950821> [retrieved on 20171018]

## Description

### FIELD OF INVENTION

This invention relates to measurement of the thickness of a retina.

### BACKGROUND

Retinal thickness in the central retina is frequently used to detect diseases in their early stages, and to monitor the effectiveness of treatment. Generally, images and data obtained with optical coherence tomography (OCT) systems are used. A standard spectral OCT system typically splits light from a single source into two parts, each of which traverses a different path in an interferometer. One path, called the reference path, simply introduces a variable delay into the beam travelling the reference path. The other path, called the object path, travels to and scatters back from a patient's eye. The light scattered back from the patient's eye is mixed with light from the reference path to produce an interference signal. The interference signal is analyzed with a spectrometer. For a usable OCT signal to be produced the two paths must be matched in length. However, this means that patient movement tends to be an issue. Complicated image processing must be performed in order to compensate for patient movement when determining the retinal thickness when using a standard OCT system.
WO 2016/004508 A1 describes a method for determining the thickness of a retina. *"*In vivo human retinal imaging by Fourier domain optical coherence tomography" authored by Wojtkowski et al. describes obtaining tomograms of human retina by Fourier domain optical coherence tomography.

There is a need to provide a method of measuring retinal thickness using a simpler procedure, and which preferably does not depend on lack of movement by the patient.

### SUMMARY

According to one embodiment of the invention, a method is provided for determining the thickness of a retina according to claim 1. According to another embodiment of the invention, a system is provided for determining the thickness of the retina according to claim 4.

By using the autocorrelation signal within a reflected beam to determine the thickness of a patient's retina, movement by the patient need not be compensated for. Diagnosis of various retinal diseases is thereby simpler and more effective.

### BRIEF DESCRIPTION OF THE DRAWINGS

The features and advantages of embodiments of the invention will become more apparent from the following detailed description of the preferred embodiment(s) with reference to the attached figures, wherein:
FIG. 1 shows a flowchart of a method by which the thickness of a retina is determined according to one embodiment of the invention;
FIG. 2a shows an example plot of the power of an autocorrelation signal versus spatial dimension for a sample reflected signal is shown according to one embodiment of the invention;
FIG. 2b shows the plot of FIG. 2a in which the power of the autocorrelation signal is represented logarithmically;
FIG. 3 shows a flowchart of a method by which the thickness of a retina is determined using an OCT system not according to one embodiment of the invention and
FIG. 4 shows an example photograph of the intensity of the autocorrelation signal as a function of position across the retina and depth within the eye.

It is noted that in the attached figures, like features bear similar labels.

### DETAILED DESCRIPTION OF THE PREFERRED EMBODIMENTS

The autocorrelation signal within a beam of light reflected by a patient's retina is produced by different reflections of a transmitted beam, the transmitted beam reflecting off different layers of the retina and interfering with each other. The dominant scatterers in the retina are the nerve fiber layer (NFL) and the layers between the inner segment/outer segment (IS/OS) and the retinal pigment epithelium (RPE). Although mixing occurs between all layers, the dominant signal is produced by interference between light reflected off these two regions. Since reflections from each component are encoded in the same beam at the same time because of autocorrelation, movement by the patient is not an issue. This simplifies image processing.

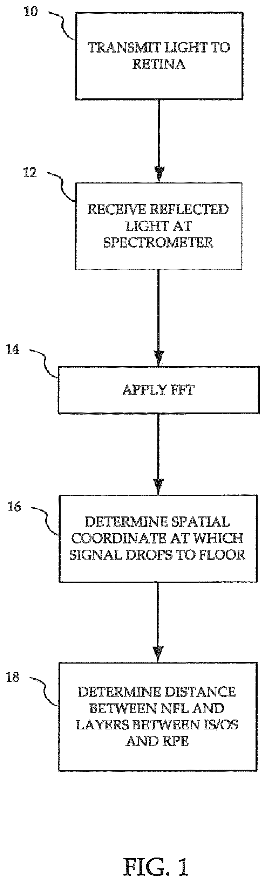

Referring to FIG. 1, a flowchart of a method of determining the thickness of a retina according to one embodiment of the invention is shown. At step 10 a beam of light is transmitted towards a patient's retina. At step 12 a reflected beam from the patient's retina is received at a spectrometer. The step 12 of receiving a reflected beam from the patient's retina is carried out over an integration time.

The spectrometer generates a frequency spectrum signal from the beam received at step 12. The frequency spectrum generated by the spectrometer contains multiple frequencies due to the distributed signals in the received beam, which in turn are due to distributed scattering by features throughout the depth of the retina. At step 14 a processor applies a Fast Fourier Transform (FFT) to the frequency spectrum signal and generates a spatial domain signal.

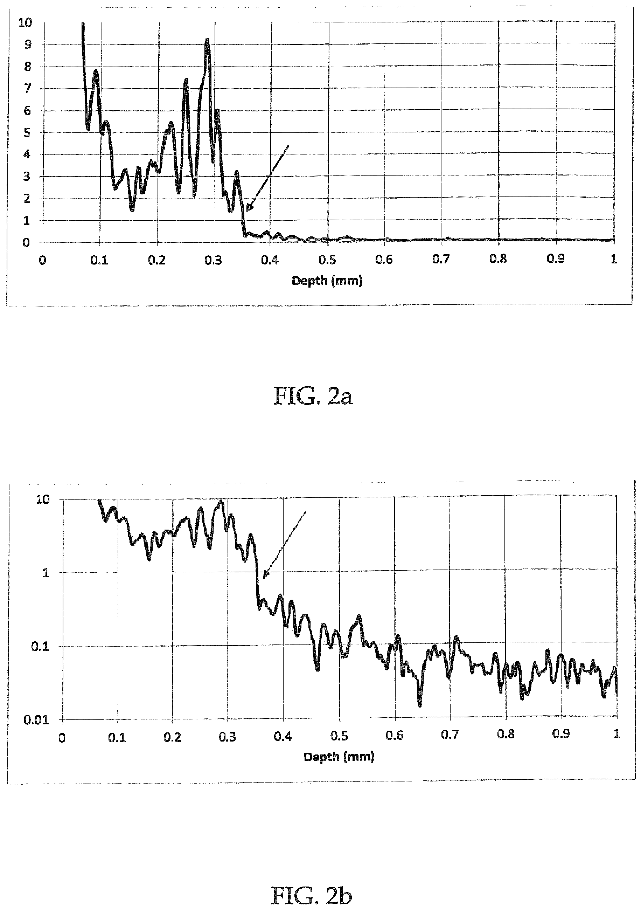

At step 16 the processor determines the spatial coordinate at which the power of the spatial domain signal drops to a floor of the power of the signal. A sample plot is shown in FIG. 2a and 2b to better illustrate this. In each figure, the spatial coordinate of the spatial domain signal (the horizontal axis of the plot) produced at step 14 has been converted into units representing the distance relative to the retina. The vertical axis indicates the power of the spatial domain signal. As can be seen, between 0.00 mm and about 0.35 mm the spatial domain signal has significant power, due to autocorrelation of the signal reflected by the retina. At distances greater than about 0.35 mm there is very little power within the spatial domain signal. The low power beyond about 0.35 mm can viewed as the floor of the power of the signal. In FIG. 2a the vertical axis of the plot shows the power of the spatial domain signal. FIG. 2b illustrates the same plot where the vertical axis represents the logarithm of the power of the spatial domain signal. In this representation of the power of the signal, the drop to the floor can be more clearly seen as occurring at the distance at which there is the sharpest drop in the logarithm of the power.

The spatial coordinate at which the power of the spatial domain signal drops to the floor of the signal can be determined using edge detection techniques, or other signal processing techniques such as, but not limited to, threshold detection.

The sharp drop in power occurs for a depth at which there stops being significant autocorrelation within the signal reflected from the retina. As explained above, this occurs when there is no interference between the signal reflected from the NFL and the signal reflected from the layers between the IS/OS and the RPE. Once the processor determines the spatial coordinate at which this sharp drop occurs, then at step 18 the distance between the NFL and the layers between the IS/OS and the RPE can be determined, and the thickness of the retina deduced.

Any of a number of different systems can be used to carry out the method described above with reference to FIG. 1. Because the interference is between different reflections within the same beam, an interference pattern does not require interference with a separate beam. In one embodiment not covered by the claims, an optical coherence tomography (OCT) system is used to measure the thickness of a retina. In an OCT system, an OCT signal is produced by interference between a reference beam and a reflected beam, the reflected beam being the reflection of an object beam off a patient's retina. The autocorrelation signal is present in the reflected beam reaching the interferometer. The autocorrelation signal is usually considered noise, and OCT systems are normally designed to suppress the autocorrelation signal relative to the OCT signal by suitable choice of reference beam power. This is possible because the OCT signal is a function of the reference beam power and the object beam power, whereas the autocorrelation signal is a function only of the object beam power. However according to this embodiment of the invention, measurement of the autocorrelation signal in the object beam is desired, and there is no need, or even desire, to suppress the autocorrelation signal.

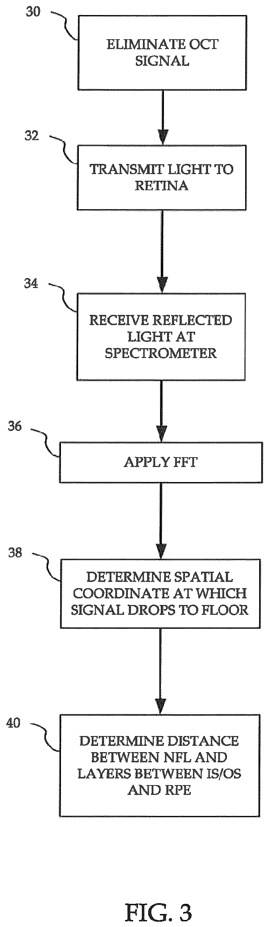

Referring to FIG. 3, a flowchart of a method not covered by the claims of determining the thickness of a retina using an OCT system according to one embodiment of the invention is shown. At step 30 the possibility of an OCT signal is eliminated, so that the only signal reaching the spectrometer of the OCT system will be the reflected object arm beam. The OCT signal is most easily eliminated by setting the reference path to an extreme position, such as at a maximum or a minimum length, resulting in no interference between the reference beam and the reflected beam within the object path. However other means of eliminating an OCT signal may be used, such as by attenuating the reference arm power.

At step 32 a beam of light is transmitted towards a patient's retina along the object arm of the OCT system. At step 34 a reflected beam from the patient's retina is received at a spectrometer. Normally this beam would result from interference between a signal in the reference arm and a signal in the object arm, the latter being reflected from the patient's retina. However, since the OCT signal has been eliminated at step 32, such as by extending the reference path to an extreme position, the only beam received by the spectrometer is the reflected beam within the object arm, and the autocorrelation signal is visible as more than just noise.

The spectrometer generates a frequency spectrum signal from the beam received at step 34. The frequency spectrum generated by the spectrometer contains multiple frequencies due to the distributed signals in the received beam, which in turn is due to distributed scattering by features throughout the depth of the retina. At step 36 a processor applies a Fast Fourier Transform (FFT) to the frequency spectrum signal and generates a spatial domain signal. At step 38 the processor locates the spatial coordinate of the spatial domain signal at which the power of the spatial domain signal drops to a floor of the power of the signal. As explained above, a sharp edge arises because of interferometry between the signal reflected from the NFL and the signal reflected from the layers between the IS/OS and the RPE. Once this spatial coordinate is located, then at step 40 the distance between the NFL and the layers between the IS/OS and the RPE can be determined, and the thickness of the retina deduced.

In another embodiment not covered by the claims, a scanning laser ophthalmoscopy (SLO) system is used to measure the thickness of a retina. The avalanche photodiode (APD) or photomultiplier tube (PMT) normally found in an SLO system is replaced with a spectrometer in order to produce a frequency spectrum of the beam reflected from a patient's retina. Alternatively, the APD or PMT can be kept in place in order that the SLO functions can still be used, but part of the returned beam is split and sent to a spectrometer in order to produce a frequency spectrum of the reflected beam. Unlike an OCT system, there is no reference beam contributing to the beam received by the spectrometer and the spectrometer used in such an embodiment needs to be more sensitive than that used in an OCT system. However, this greater sensitivity can be achieved with longer integration times and much less expensive charge-coupled devices (CCDs) or complementary metal-oxide-semiconductor (CMOS) arrays than are used in OCT systems. The step of receiving a reflected beam from the patient's retina is carried out over this longer integration time. A processor then determines the thickness of the retina from the reflected beam as described above.

In yet another embodiment not covered by the claims, a fixed point measurement system is used to measure the thickness of a retina. The high sensitivity requirement of the spectrometer required for clear detection of the autocorrelation signal can be achieved with use of a 2D camera in the spectrometer, such as a low cost video CCD, in order to analyze light returned from the fixed point.

The retinal thickness is determined using a line scan system. A 2D-camera is employed in the spectrometer. According to the invention, a beam is transmitted towards an eye, and then optical components smear out the transmitted beam so that it hits the retina along a line across the retina. The signal is reflected from multiple fixed points on the retina at the same time. A plurality of frequency spectra is produced, each frequency spectra corresponding to one point from the line across the retina. An FFT is applied to each frequency spectra, thereby producing a plurality of spatial domain signals (SDSs). A plurality of spatial measurements is determined, each spatial measurement being that at which the power of a corresponding one of the SDSs falls to a floor power of the SDS. Such spatial measurements can be determined using edge detection techniques, or other signal processing techniques such as, but not limited to, threshold detection. In short, the method described above with reference to FIG. 1 is carried out in parallel by the processor, once for each point imaged by the line scan system.

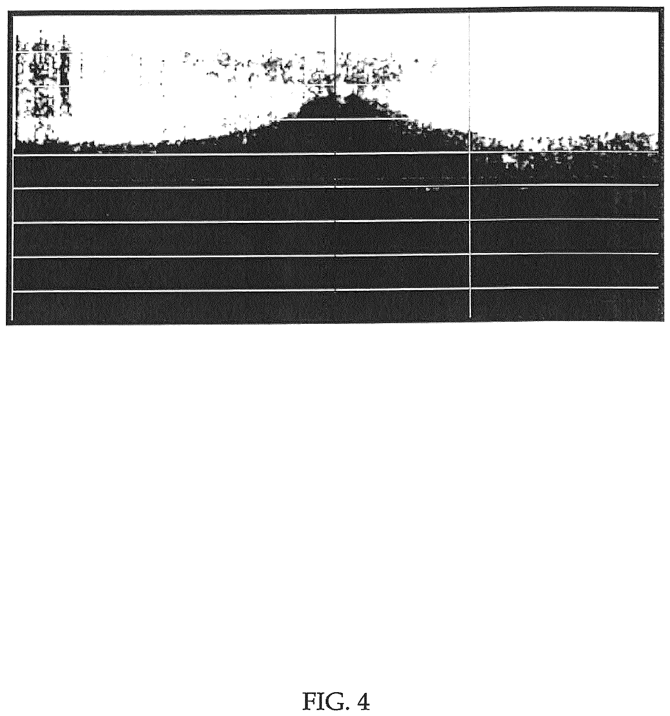

Alternatively, the plurality of spatial domain signals can be used to generate a two-dimensional image, an example of which is shown in FIG. 4.

In FIG. 4, the power of the spatial domain signal for a given position across the retina is indicated by the intensity of the plot, the brightness of the diagram indicating the intensity. The horizontal axis indicates the position along a line of the retina. The vertical axis indicates the depth within the retina, and corresponds generally with the horizontal axis shown in FIG. 2a and FIG. 2b. Edge detection techniques, or other signal processing techniques such as, but not limited to, threshold detection, can be used to locate the distance at which the signal drops sharply, as in the signal dimension signal shown in FIG. 2a and FIG. 2b, but the edge is even more apparent in the two-dimensional image shown in FIG. 4 as the edge between the light and dark regions. A thickness measure can then be determined for each of a plurality of points in the image as the distance between the located edge and a fixed edge for the point at which there is significant power in the SDS, e.g. the top edge of the data set shown in FIG. 4.

In either case, the "thickness of the retina" can comprise a plurality of thickness measures, one for each point along across the retina, a plurality of thickness measures determined only for a subset of these points, thickness measure for only one of these points, or a collective measure based on the statistics of these measures (e.g. their average).

A thickness map can also be generated by determining thicknesses of the retina across multiple lines across the retina.

In the embodiments described above, the thickness of the retina is derived from an autocorrelation signal using a spectrometer. Alternatively, regular interferometry and variable path imbalance can be used to measure the thickness of the retina directly. As yet another alternative, a tunable laser and the Fourier domain can be used.

The methods described above for determining the thickness of a retina can be used in methods for screening and diagnosing of various retina and ocular diseases not covered by the claims. Such diseases include diabetic retinopathy, diabetic macular edema, age-related macular degeneration (AMD), cystoid macular edema, central serous retinopathy, central retinal vein occlusion, central retinal artery occlusion, and glaucoma. Retinal thickness increases dramatically in such diseases due to death of cells of specific inner or outer layers of the retina due to the above diseases at more progressive stages. Accordingly, in one embodiment of the invention the methods described above for determining the thickness of a retina are preliminary steps in a method for screening and/or diagnosing such diseases.

The logic of the methods described above may be stored as instructions stored on a non-transitory computer-readable storage medium in a form executable by a computer processor, although in the embodiment in which an OCT system is used to carry out the method the elimination the OCT signal may be carried out manually instead. The logic of the methods described above may also be stored as instructions within a memory accessible by a processor, such that when read and executed by the processor they cause the processor to carry out the methods. The processor may be implemented by a general purpose processor, a network processor, a digital signal processor, an ASIC, or multiple such devices.

The embodiments presented are exemplary only and persons skilled in the art would appreciate that variations to the embodiments described above may be made without departing from the scope of the invention defined solely by the appended claims.

## Claims

1. A method of determining the thickness of a retina, comprising:
illuminating (10, 32) the retina with a line of light that hits the retina along a line across the retina;
receiving (12, 34) a reflected line of light, the reflected line of light having been reflected from multiple fixed points on the retina at the same time;
producing a plurality of frequency spectra from the reflected line of light, one frequency spectrum for each of a plurality of points within the reflected line of light, each of the plurality of points within the reflected line of light corresponding to one of a plurality of points in the retina;
applying (14, 36) a Fast Fourier Transform**,** FFT, separately to each of the plurality of frequency spectra to produce a plurality of spatial domain signals**,** SDSs, each one of the plurality of SDSs corresponding to one of the plurality of frequency spectra; and
determining (18, 40) the thickness of the retina at each of the plurality of points across the retina, the thickness of the retina at each of the plurality of points being determined (16, 38) from a spatial coordinate of the SDSs at which the power of the SDS corresponding to the point in the retina drops to a floor power of the SDS.

2. The method of claim 1 wherein determining the thickness of the retina comprises:
determining (18, 40) a distance between a nerve fiber layer**,** NFL, and the layers between the inner segment/outer segment, IS/OS, and the retinal pigment epithelium**,** RPE, from the spatial coordinate of the SDS at which the power of the SDS drops to the floor power; and
deducing the thickness of the retina from the determined distance between the NFL and the layers between the IS/OS and the RPE.

3. The method of claim 1 or claim 2 further comprising eliminating (30) the possibility of interference between a signal in a reference arm of an optical coherence tomography**,** OCT, system and a signal in an object arm of the OCT system, and wherein the line of light used to illuminate the retina is the signal in the object arm of the OCT system.

4. A system for determining the thickness of a retina, comprising:
a light source configured to generate a beam of light;
optical components configured to shape the beam of light into a line of light that hits the retina along a line across the retina, the reflected line of light having been reflected from multiple fixed points on the retina at the same time;
a spectrometer configured to receive a reflected line of light; and
a processor operably coupled to the spectrometer, the processor configured carry out the method of any one of claims 1 to 3.

## Patentansprüche

1. Verfahren zur Bestimmung der Dicke einer Netzhaut, umfassend:
Beleuchten (10, 32) der Netzhaut mit einer Lichtlinie, die entlang einer Linie quer über die Netzhaut auf die Netzhaut trifft;
Empfangen (12, 34) einer reflektierten Lichtlinie, wobei die reflektierte Lichtlinie gleichzeitig von mehreren festen Punkten auf der Netzhaut reflektiert wurde;
Erzeugen mehrerer Frequenzspektren aus der reflektierten Lichtlinie, wobei für jeden der mehreren Punkte innerhalb der reflektierten Lichtlinie ein Frequenzspektrum erzeugt wird, wobei jeder der mehreren Punkte innerhalb der reflektierten Lichtlinie einem der mehreren Punkte auf der Netzhaut entspricht;
Anwenden (14, 36) einer schnellen Fourier-Transformation, FFT, separat auf jedes der mehreren Frequenzspektren, um mehrere Ortsbereichssignale, SDS, zu erzeugen, wobei jedes der mehreren SDS einem der mehreren Frequenzspektren entspricht; und
Bestimmen (18, 40) der Dicke der Netzhaut an jedem der mehreren Punkte auf der Netzhaut, wobei die Dicke der Netzhaut an jedem der mehreren Punkte aus einer räumlichen Koordinate der SDS bestimmt wird (16, 38), bei der die Leistung des SDS, das dem Punkt in der Netzhaut entspricht, auf eine Mindestleistung des SDS abfällt.

2. Verfahren nach Anspruch 1, wobei Bestimmen der Dicke der Netzhaut Folgendes umfasst:
Bestimmen (18, 40) eines Abstands zwischen einer Nervenfaserschicht, NFL, und den Schichten zwischen dem inneren Segment/äußeren Segment, IS/OS, und dem retinalen Pigmentepithel, RPE, aus der räumlichen Koordinate des SDS, bei der die Leistung des SDS auf den Minimalwert abfällt; und
Ableiten der Dicke der Netzhaut aus dem bestimmten Abstand zwischen der Nervenfaserschicht NFL und den Schichten zwischen dem IS/OS und dem RPE.

3. Verfahren nach Anspruch 1 oder 2, ferner umfassend Beseitigen (30) der Möglichkeit einer Interferenz zwischen einem Signal in einem Referenzarm eines optischen Kohärenztomographie-Systems, OCT-Systems, und einem Signal in einem Objektarm des OCT-Systems, und wobei die zur Beleuchtung der Netzhaut verwendete Lichtlinie das Signal im Objektarm des OCT-Systems ist.

4. System zur Bestimmung der Dicke einer Netzhaut, umfassend:
eine Lichtquelle, die so konfiguriert ist, dass sie einen Lichtstrahl erzeugt;
optische Komponenten, die so konfiguriert sind, dass sie den Lichtstrahl in eine Lichtlinie formen, die entlang einer Linie quer über die Netzhaut auf die Netzhaut trifft, wobei die reflektierte Lichtlinie gleichzeitig von mehreren festen Punkten auf der Netzhaut reflektiert wurde;
ein Spektrometer, das so konfiguriert ist, dass es eine reflektierte Lichtlinie empfängt; und
einen Prozessor, der mit dem Spektrometer funktionsfähig gekoppelt ist, wobei der Prozessor so konfiguriert ist, dass er das Verfahren nach einem der Ansprüche 1 bis 3 durchführt.

## Revendications

1. Procédé de détermination de l'épaisseur de la rétine, comprenant :
l'éclairage (10, 32) de la rétine avec un trait de lumière qui atteint la rétine suivant une ligne traversant la rétine ;
la réception (12, 34) d'un trait de lumière réfléchi, le trait de lumière réfléchi ayant été réfléchi, simultanément, à partir de plusieurs points fixes situés sur la rétine ;
la production d'une pluralité de spectres de fréquences à partir du trait de lumière réfléchi, un spectre de fréquence pour chacun parmi une pluralité de points situés dans le trait de lumière réfléchi, chacun parmi la pluralité de points situés dans le trait de lumière réfléchi correspondant à l'un parmi la pluralité de points situés dans la rétine ;
l'application (14, 36) d'une transformée de Fourier rapide (FFT) séparément à chacun parmi la pluralité de spectres de fréquences pour produire une pluralité de signaux du domaine spatial (SDS), chacun parmi la pluralité de SDS correspondant à l'un parmi la pluralité de spectres de fréquences ; et
la détermination (18, 40) de l'épaisseur de la rétine au niveau de chacun parmi la pluralité de points sur la rétine, l'épaisseur de la rétine au niveau de chacun parmi la pluralité de points étant déterminée (16, 38) à partir d'une coordonnée spatiale des SDS à laquelle la puissance du SDS correspondant au point de la rétine chute jusqu'à une puissance minimale du SDS.

2. Procédé selon la revendication 1, dans lequel la détermination de l'épaisseur de la rétine comprend :
la détermination (18, 40) d'une distance entre une couche de fibres nerveuses (NFL) et les couches situées entre le segment interne/segment externe, l'IS/l'OS et l'épithélium pigmentaire rétinien (RPE), à partir de la coordonnée spatiale du SDS à laquelle la puissance du SDS chute jusqu'à la puissance minimale ; et
la déduction de l'épaisseur de la rétine à partir de la distance déterminée entre la NFL et les couches situées entre l'IS/l'OS et le RPE.

3. Procédé selon la revendication 1 ou la revendication 2 comprenant en outre l'élimination (30) de la possibilité d'interférence entre un signal dans un bras de référence d'un système de tomographie par cohérence optique (OCT) et un signal dans un bras objet du système OCT, et dans lequel le trait de lumière utilisé pour éclairer la rétine est le signal dans le bras objet du système OCT.

4. Système permettant de déterminer l'épaisseur de la rétine, comprenant :
une source de lumière configurée pour générer un faisceau lumineux ;
des composants optiques configurés pour façonner le faisceau lumineux en un trait de lumière qui atteint la rétine suivant une ligne traversant la rétine, le trait de lumière réfléchi ayant été réfléchi, simultanément, par plusieurs points fixes situés sur la rétine ;
un spectromètre configuré pour recevoir un trait de lumière réfléchi ; et
un processeur couplé de manière fonctionnelle au spectromètre, le processeur étant configuré pour mettre en œuvre le procédé selon l'une quelconque des revendications 1 à 3.
